# EUROPÄISCHE PATENTSCHRIFT

(11) **EP 2 394 567 B1**
(45) Veröffentlichungstag und Bekanntmachung des Hinweises auf die Patenterteilung: **06.12.2017**
(21) Anmeldenummer: 11169262.0
(22) Anmeldetag: 09.06.2011
(51) Int. Cl.: F28D 15/06, A61B 1/00, A61B 1/12, A61B 1/06, G02B 23/24

(54) **Endoskop**
Endoscope
Endoscope

(30) Priorität: 11.06.2010 DE 102010024003
(43) Veröffentlichungstag der Anmeldung: 14.12.2011
(73) Patentinhaber: Karl Storz GmbH & Co. KG, 78532 Tuttlingen (DE)
(72) Erfinder: Schrader, Stephan, 14532 Kleinmachnow (DE); Oginski, Stefan, 10369 Berlin (DE); Brüggemann, Daniel, 10551 Berlin (DE)
(74) Vertreter: Witte, Weller & Partner Patentanwälte mbB

(56) Entgegenhaltungen:
- EP-A1- 1 731 862
- EP-A1- 2 311 366
- WO-A2-2004/011848
- DE-A1-102009 049 196
- US-A1- 2008 151 046
- US-A1- 2008 208 006
- US-A1- 2008 208 297
- US-A1- 2010 003 633

## Beschreibung

Die Erfindung betrifft ein Endoskop, mit einem langerstreckten Schaft, mit einem Kopfstück an einem proximalen Ende des Schafts, wobei das Kopfstück ein Gehäuse aufweist, mit zumindest einer in dem Schaft in einem distalen Bereich desselben angeordneten Lichtquelle, die Verlustwärme erzeugt, und mit einer passiven Kühlung, die zumindest ein in dem Schaft angeordnetes Wärmerohr in Form einer Heatpipe aufweist, die mit der zumindest einen Lichtquelle thermisch gekoppelt ist, um die Verlustwärme in Richtung nach proximal abzuführen, wobei sich das zumindest eine Wärmerohr bis in das Kopfstück erstreckt, und wobei in dem Kopfstück ein Wärmesenkekörper angeordnet ist, mit dem das zumindest eine Wärmerohr thermisch gekoppelt ist, und der die Verlustwärme von dem zumindest einen Wärmerohr aufnimmt und direkt oder über das Gehäuse des Kopfstücks an die Umgebung abgibt.

Ein solches Endoskop ist aus dem Dokument US 2008/208297 A1 bekannt.

In dem vorstehend genannten Dokument ist eine optische Therapieeinrichtung offenbart, die ein Handstück und ein Einführrohr zum Einführen in einen Körperhohlraum aufweist. Eine Lichtquelle ist am distalen Ende des Einführrohrs angeordnet, wobei die Lichtquelle aus LEDs gebildet sein kann. Von den LEDs erzeugte Wärme wird mittels Heatpipes in das Handstück bis zu einer Wärmesenke, die am proximalen Ende des Handstücks angeordnet ist, abgeführt.

In dem Dokument US 2008/0151046 A1 ist ein technisches Endoskop beschrieben, das zur Untersuchung von Maschinen, insbesondere Flugzeugtriebwerken, verwendet wird. In dem Dokument DE 10 2009 049196 A1 sind zwei Wärmerohren die sich nacheinander erstrecken und drehbar sind beschreiben. Das Dokument bezieht sich aber auf ein Fahrzeug und nicht ein Endoskop.

Die vorliegende Erfindung betrifft insbesondere ein medizinisches Endoskop, das im Rahmen der minimal-invasiven Chirurgie als Beobachtungsinstrument verwendet wird. Bei einem solchen medizinischen Endoskop wird der langerstreckte Schaft durch eine künstlich geschaffene oder natürliche Öffnung teilweise in den Körper eingeführt.

Heutzutage weisen Endoskope eine oder mehrere in den Endoskopschaft integrierte Lichtquellen auf, die als Leuchtdioden (LED) ausgebildet sind. Durch die Integration einer oder mehrerer Lichtquellen in den Schaft des Endoskops, üblicherweise im distalen Bereich des Schafts, kann auf eine externe Lichtquelle, beispielsweise eine Xenon-Lampe, verzichtet werden. Der Vorteil einer oder mehrerer in den Schaft des Endoskops integrierter Lichtquellen besteht darin, dass das bei Verwendung einer externen Lichtquelle erforderliche Lichtleitkabel wegfällt, wodurch die Ergonomie des Endoskops verbessert ist. Durch den Wegfall externer Lichtquellen und Lichtleitkabel werden außerdem die Herstellungs- und Anschaffungskosten von Endoskopen reduziert.

Bei einem Endoskop, das eine in den Schaft integrierte Lichtquelle aufweist, ergibt sich jedoch das technische Problem, dass die Lichtquelle im Betrieb an ihrem Ort Verlustwärme in dem Schaft erzeugt, die den Schaft mitunter stark erwärmt, insbesondere im Bereich des distalen Endes des Schafts, wo die Lichtquelle(n) angeordnet ist (sind).

Ein Endoskop, das in der Chirurgie verwendet wird, unterliegt Vorschriften des Medizinproduktgesetzes. Gemäß dem Medizinproduktgesetz darf der Endoskopschaft außenseitig eine Temperatur von 41°C nicht überschreiten, um eine wärmebedingte Beschädigung von Gewebe im menschlichen oder tierischen Körper zu vermeiden.

Heutige Hochleistungs-LEDs erzeugen jedoch so viel Verlustwärme, dass sich der Schaft über die geforderte Maximaltemperatur von 41°C hinaus rasch erwärmen würde. Vor diesem Hintergrund ist es erforderlich, in dem Schaft eine Kühlung vorzusehen, die die Verlustwärme so abführt, dass sich der Schaft nicht über die gesetzlich vorgeschriebene Maximaltemperatur hinaus erwärmt.

Zur Kühlung bzw. Abfuhr der Verlustwärme sind verschiedene Konzepte bekannt.

Bei dem aus dem Dokument US 2008/0151046 A1 bekannten Endoskop ist zur Abfuhr der Verlustwärme von der im distalen Bereich des Schafts angeordneten Lichtquelle, die dort als LED ausgebildet ist, ein Wärmerohr in Form einer Heatpipe vorhanden, dessen distales Ende mit der Lichtquelle thermisch leitend gekoppelt ist. Ein solches Wärmerohr ist ein Wärmeüberträger, der unter Nutzung von Verdampfungswärme eines Stoffes, der in dem Wärmerohr enthalten ist, eine hohe Wärmestromdichte erlaubt, d.h. auf kleiner Querschnittsfläche können große Mengen Wärme transportiert werden. Zur Umwälzung des als Wärmetransportmedium in dem Wärmerohr vorhandenen Stoffes wird keine zusätzliche Hilfsenergie wie beispielsweise eine Umwälzpumpe benötigt, wodurch sich der Wartungsaufwand und die Betriebskosten eines solchen Wärmerohrs minimieren.

Eine spezielle Art eines Wärmerohrs ist die sogenannte Heatpipe. Bei einer Heatpipe verdampft das Wärmeträgermedium am "warmen" Ende des Wärmerohrs, wobei der Dampf zum "kalten" Ende geleitet wird, wo der Dampf wieder kondensiert. Bei der Wärmeabfuhr wird somit latente Wärme genutzt, wodurch die Wärmeleitung in einer Heatpipe gegenüber einer Wärmeleitung in beispielsweise Kupfer um das Zig-fache größer ist. In der Heatpipe wird Flüssigkeit, die als Wärmetransportmedium dient, mittels Kapillaren nach dem Dochtprinzip zum Verdampfungsende zurückgeführt. Das kondensierte Fluid fließt daher lageunabhängig in der Kapillare zurück zum Verdampfungsende des Wärmerohrs, d.h. dem mit der Lichtquelle gekoppelten Ende des Wärmerohrs. Aufgrund der Ausnutzung des Kapillarprinzips arbeiten Heatpipes daher unter beliebigen Orientierungen zur Schwerkraft.

Bei dem aus US 2008/0151046 A1 bekannten Endoskop erstreckt sich das Wärmerohr über eine kleine Länge des Schafts des Endoskops nach proximal, und mit dem proximalen Ende des Wärmerohrs ist ein wärmeleitfähiger Draht, beispielsweise ein Kupferdraht verbunden, wobei der Draht sich weiter nach proximal erstreckt und mit seinem proximalen Ende mit der Innenseite des Schaftes des Endoskops wärmeleitend verbunden ist.

Diese Art der Kühlung ist für ein medizinisches Endoskop jedoch unzureichend, da die Wärme von dem Wärmerohr auf den Schaft des Endoskops übertragen wird, wodurch sich der Schaft erwärmt, was jedoch bei einem medizinischen Endoskop gerade vermieden werden soll.

Aus DE 296 13 103 U1 ist ein Endoskop für medizinische Zwecke bekannt, in dessen Schaft distalseitig lichtemittierende Elemente angeordnet sind, wobei in dem Schaft Kanäle zum Hindurchleiten eines Fluids zur Kühlung vorhanden sind.

Aus JP 2007 007 321 A ist ein Endoskop bekannt, in dessen Schaft im distalen Bereich eine Lichtquelle, die als LED ausgebildet ist, angeordnet ist, wobei zur Kühlung bzw. Abfuhr der Verlustwärme der LED in dem Schaft eine Rohrleitung verlegt ist, durch die Luft zur Kühlung hindurchgeleitet wird.

US 2008/0208006 A1 offenbart ein Endoskop mit einem langgestreckten Schaft und einem Kopfstück an einem proximalen Ende des Schafts und einer an einem distalen Bereich des Schafts angeordneten Lichtquelle, die zur Abfuhr der von der Lichtquelle erzeugten Abwärme mit einem Wärmerohr in Form einer Heatpipe verbunden ist, die dazu vorgesehen ist, die durch die Lichtquelle erzeugte Abwärme nach proximal abzuführen.

EP 1 731 862 A1 offenbart eine Lichtquelle für ein Endoskop, wobei die Lichtquelle einen Kühlkörper aufweist, der mit der Lichtquelle thermisch gekoppelt ist. Es ist zudem eine Heatpipe vorgesehen, die die von der Lichtquelle an den Kühlkörper abgegebene Wärme ableitet. Die Lichtquelle ist zudem dazu vorgesehen, das Licht am proximalen Ende eines Endoskopschafts einzukoppeln.

Weitere Endoskope, bei denen die Kühlung der im Schaft angeordneten Lichtquelle mittels eines externen Kühlkreislaufes bewerkstelligt wird, sind aus JP 2007 229 261 A, JP 2007 007 322 und EP 1 738 679 A2 bekannt.

WO 2004/011848 A2 offenbart ein Instrument zum Aushärten von lichtaushärtbaren Materialien. Dieses Instrument weist einen rohrförmigen Schaft aus Kunststoff oder Metall auf. Zum Aushärten der Materialien ist eine LED am distalen Ende des Schafts angeordnet. Zur Abfuhr der durch die LED erzeugten Wärme ist zudem eine Heatpipe vorgesehen, die sich durch den Schaft erstreckt und mittels Klebstoff oder Lot mit der LED verbunden ist. Die Heatpipe erstreckt sich bis zu einer am proximalen Ende des Schafts angeordneten Wärmesenke aus Aluminium oder Kupfer, mit der die Heatpipe fest verbunden ist.

Bei den vorstehend genannten bekannten Kühlkonzepten erfolgt die Kühlung des Endoskopschafts somit mittels eines externen Kühlkreislaufes, wobei ein Kühlmedium, beispielsweise ein gasförmiges oder flüssiges Kühlmedium, durch den Schaft geleitet wird.

Der Nachteil eines solchen Kühlkonzepts besteht jedoch darin, dass für den Kühlkreislauf beispielsweise externe Kühlleitungen, Umwälzpumpen und ein Reservoir für das Kühlmedium erforderlich sind, was zum einen den Herstellungs- und Kostenaufwand eines derartigen Endoskops erhöht, und zum anderen auch die Ergonomie des Endoskops beeinträchtigt, weil das Endoskop mit externen Kühlleitungen des Kühlkreislaufes verbunden werden muss, die bei der Handhabung des Endoskops in einem chirurgischen Eingriff störend wirken.

Es ist eine Aufgabe der vorliegenden Erfindung, ein Endoskop der eingangs genannten Art dahingehend weiterzubilden, dass mit geringem Herstellungs- und Kostenaufwand eine zuverlässige Abfuhr der Verlustwärme von der zumindest einen im Schaft des Endoskops angeordneten Lichtquelle sowie eine erhöhte Funktionalität des Endoskops erreicht wird.

Erfindungsgemäß wird diese Aufgabe hinsichtlich des eingangs und in Anspruch 1 genannten Endoskops dadurch gelöst, dass das zumindest eine erste Wärmerohr ein erstes Wärmerohr, das mit der zumindest einer Lichtquelle gekoppelt ist und sich bis zu einem Zwischenbereich im Schaft zwischen dem distalen und proximalen Ende desselben erstreckt, und zumindest ein zweites Rohr in Form einer Heatpipe aufweist, das mit dem ersten Wärmerohr thermisch gekoppelt ist, und das sich von dem Zwischenbereich bis in das Kopfstück erstreckt und mit dem Wärmesenkekörper thermisch gekoppelt ist, und dass das zumindest eine zweite Wärmerohr relativ zum Schaft um eine Längsachse des zumindest einen zweiten Wärmerohrs drehbar ist, und dass das zumindest eine zweite Wärmerohr einerseits mit einer im distalen Bereich des Schafts angeordneten Kamera verbunden und andererseits mit einem Drehantrieb verbunden ist, um eine Drehung der Kamera relativ um ihre eigene optische Achse relativ zum Schaft zu ermöglichen.

Das erfindungsgemäße Endoskop nutzt somit für die Abfuhr der von der zumindest einen Lichtquelle im distalen Bereich des Schafts erzeugte Verlustwärme die Vorteile einer Heatpipe, wodurch ein Kühlkreislauf und damit Anschlüsse für die Zufuhr eines Kühlmediums und Anschlüsse für die Abfuhr eines Kühlmediums und externe Kühlleitungen vermieden werden. Dadurch, dass sich das zumindest eine Wärmerohr bis in das Kopfstück erstreckt, in dem ein Wärmesenkekörper angeordnet ist, wird die Verlustwärme mit hoher Übertragungsrate bis zum Kopfstück transportiert, wo die Wärme an das großflächige Gehäuse des Kopfstückes, also an eine große Fläche und über diese oder direkt an die Umgebung abgegeben wird. Im letzteren Fall kann der Wärmesenkekörper selbst ein Teil des Gehäuses des Kopfstücks sein. Da das Kopfstück bei einem medizinischen Endoskop während eines chirurgischen Eingriffs außerhalb des menschlichen oder tierischen Körpers angeordnet ist, führt die Wärmeabgabe über das Kopfstück an die Umgebung nicht zu einer wärmebedingten Schädigung von Gewebe innerhalb des menschlichen oder tierischen Körpers. Der Schaft des Endoskops wird bei dem erfindungsgemäßen Endoskop somit hinreichend gegen eine Erwärmung geschützt, so dass das erfindungsgemäße Endoskop den gesetzlichen Anforderungen an die Maximaltemperatur des Schafts erfüllt. Das im Verhältnis zum Endoskopschaft großflächigere Kopfstück ist in der Lage, die Wärme vom Wärmekörper, der ebenfalls großflächig ist, gut aufzunehmen und an die Umgebung abzugeben. Durch die Bauweise des erfindungsgemäßen Endoskops mit einem Wärmerohr zur Abfuhr der von der zumindest einen Lichtquelle erzeugten Verlustwärme ist das Endoskop insgesamt sehr kostengünstig und auf konstruktiv einfache Weise herstellbar, wodurch sich das erfindungsgemäße Endoskop auch als Einwegendoskop eignen kann.

Das zumindest eine Wärmerohr weist ein erstes Wärmerohr, das mit der zumindest einen Lichtquelle gekoppelt ist und sich bis zu einem Zwischenbereich im Schaft zwischen dem distalen und proximalen Ende desselben erstreckt, und zumindest ein zweites Wärmerohr in Form einer Heatpipe auf, das mit dem ersten Wärmerohr thermisch gekoppelt ist, und das sich von dem Zwischenbereich bis in das Kopfstück erstreckt und mit dem Wärmesenkekörper thermisch gekoppelt ist.

Diese Maßnahme hat zum einen den Vorteil, dass über eine Kaskade von Wärmerohren auch Endoskope mit sehr langen Schäften mit dem erfindungsgemäßen Kühlkonzept versehen werden können. Zum anderen hat diese Maßnahme den Vorteil, dass auch Endoskope mit semi-flexiblen Schäften mit dem erfindungsgemäßen Kühlkonzept ausgestattet werden können, indem mehrere Wärmerohre in Form von Heatpipes, die jeweils für sich nur eine geringere Länge als der Schaft aufweisen, durch die Hintereinanderschaltung eine nicht-starre Anordnung ergeben.

Das zumindest eine zweite Wärmerohr ist relativ zum Schaft um eine Längsachse des zumindest einen zweiten Wärmerohrs drehbar, wobei das zumindest eine zweite Wärmerohr einerseits mit einer am distalen Bereich des Schafts angeordneten Kamera verbunden und andererseits mit einem Drehantrieb verbunden ist, um eine Drehung der Kamera um ihre optische Achse relativ zum Schaft zu ermöglichen.

In dieser Ausgestaltung ist das Endoskop ein Videoendoskop, das eine im distalen Bereich des Schafts angeordnete Kamera aufweist. Insbesondere bei Videoendoskopen mit einer sogenannten Schrägblickoptik, bei der die Blickrichtung durch das Endoskop schräg zur Längsachse des Schafts des Endoskops gerichtet ist, ist es üblicherweise vorgesehen, dass der Schaft und die Kamera relativ zueinander verdrehbar sind. Hintergrund dieser relativen Verdrehbarkeit ist, dass zur Änderung der Blickrichtung der Schaft um die eigene Längsachse gedreht wird. Um dabei den Horizont des von der Kamera aufgenommenen Bildes nicht zu verdrehen, weil andernfalls der das Endoskop bedienende Arzt seine Orientierung im Beobachtungsgebiet verlieren könnte, ist die Kamera daher relativ zum Schaft drehbar, so dass durch relative Drehung der Kamera zum Schaft das wahrgenommene Bild stets aufrecht gehalten werden kann. Die Kamera weist beispielsweise einen Bildsensor und ein diesem vorgeordnetes Objektiv auf. Soweit in der vorliegenden Anmeldung beschrieben ist, dass die Kamera drehbar ist oder gedreht wird, so ist darunter zu verstehen, dass nur der Bildsensor relativ zum Schaft drehbar ist oder gedreht wird, während das Objektiv schaftfest ist, oder dass sowohl der Bildsensor als auch das Objektiv relativ zum Schaft drehbar sind oder gedreht werden.

In der vorstehend genannten Maßnahme hat das zumindest eine zweite Wärmerohr somit vorteilhafterweise zwei Funktionen, nämlich einerseits die Abfuhr der Verlustwärme der zumindest einen Lichtquelle nach proximal, zum anderen die Übertragung des Drehmoments vom Drehantrieb auf die Kamera. Vorteilhafterweise werden hierdurch weitere Teile für den Drehantrieb der Kamera eingespart, wodurch Kosten und Herstellungsaufwand eingespart werden.

In einer bevorzugten Ausgestaltung ist der Schaft zumindest im Bereich seines distalen Endes innenseitig gegen das zumindest eine Wärmerohr thermisch isoliert.

Da sich das Wärmerohr durch die von der zumindest einen Lichtquelle aufgenommene Verlustwärme selbst erwärmt und diese Wärme zwar nach proximal abführt, wird durch die vorstehend genannte Maßnahme vorteilhafterweise vermieden, dass das Wärmerohr Wärme an den Schaft abstrahlt und dadurch der Schaft über das zulässige Maß erwärmt wird.

In dieser Ausgestaltung kann die Isolation dadurch realisiert sein, dass das Wärmerohr außenseitig mit einer Isolierung, beispielsweise einer wärmedämmenden Beschichtung versehen ist, und/oder dass der Schaft innenseitig mit einer Isolierung versehen ist, d.h. an der Innenwand des Schafts eine wärmedämmende Beschichtung aufweist, und/oder dass ein wärmedämmendes Isolierrohr zwischen der Schaftinnenwand und der Außenwand des zumindest einen Wärmerohrs angeordnet ist.

In einer weiteren bevorzugten Ausgestaltung sind elektronische und/oder elektrische Bauelemente in einem Hohlraum des Wärmesenkekörpers angeordnet.

Hierbei ist von Vorteil, dass der Wärmesenkekörper im Kopfstück des Endoskops nicht nur die vom Wärmerohr abgegebene Wärme an das Kopfstück abgibt, sondern auch die von den elektronischen oder elektrischen Bauelementen erzeugte Verlustwärme. Solche elektronischen oder elektrischen Bauelemente sind bei dem Endoskop insbesondere dann vorgesehen, wenn das Endoskop ein Videoendoskop mit einer Kamera im distalen Bereich des Schafts oder im Kopfstück ist.

In einer weiteren bevorzugten Ausgestaltung ist ein distales Ende des zumindest einen Wärmerohrs mit der zumindest einen Lichtquelle mittels eines Wärmekoppelelements thermisch leitend verbunden.

Gegenüber einer thermischen Kopplung der zumindest einen Lichtquelle mit dem zumindest einen Wärmerohr über Wärmestrahlung hat diese Maßnahme den Vorteil, dass die Wärmeübertragung von der zumindest einen Lichtquelle auf das zumindest eine Wärmerohr durch Wärmeleitung weiter verbessert ist. Ein Wärmekoppelelement kann hier ein Festkörper sein, dessen Material über eine hohe Wärmeleitfähigkeit verfügt, beispielsweise Kupfer oder Aluminium. Das Wärmekoppelelement steht dabei vorzugsweise über eine möglichst große Fläche mit der zumindest einen Lichtquelle und dem zumindest einen Wärmerohr berührend in Verbindung, ohne dass Luftspalte an diesen Berührflächen vorhanden sind, was durch Lötung, Kleben oder dergleichen bewerkstelligt sein kann.

In einer weiteren bevorzugten Ausgestaltung umgibt der Wärmesenkekörper einen proximalen Endbereich des zumindest einen Wärmerohrs und steht mit diesem unmittelbar in Berührung.

Durch diese Maßnahme werden, wie bei der Wärmeübertragung von der zumindest einen Lichtquelle auf das zumindest eine Wärmerohr mittels des zuvor genannten Wärmekoppelelements, die vorstehend genannten Vorteile einer sehr guten Wärmeübertragung auch vom proximalen Endbereich des zumindest einen Wärmerohrs auf den Wärmesenkekörper erreicht.

Es ist weiterhin bevorzugt, wenn sich das erste Wärmerohr und das zumindest eine zweite Wärmerohr in dem Zwischenbereich in Längsrichtung des Schafts teilweise überlappen.

Diese Maßnahme trägt vorteilhafterweise zu einer verbesserten Wärmeübertragung zwischen den einzelnen Wärmerohren und damit zu einer besseren Abfuhr der Verlustwärme in das Kopfstück des Endoskops bei.

In einer weiteren bevorzugten Ausgestaltung sind das erste Wärmerohr und das zumindest eine zweite Wärmerohr in dem Zwischenbereich mittels eines Wärmekoppelelements miteinander thermisch leitend verbunden, das das erste und das zumindest eine zweite Wärmerohr umgibt.

Durch diese Maßnahme wird die Wärmeübertragung in Wärmetransportrichtung noch weiter verbessert, insbesondere wenn das Wärmekoppelelement wie in einer vergleichbaren Ausgestaltung oben bereits angegeben aus einem Material besteht, das sehr gut wärmeleitend ist, beispielsweise aus Kupfer oder Aluminium.

Bei den zuvor genannten Ausgestaltungen gibt das "kalte" Ende des jeweils distalseitigen Wärmerohrs die Verlustwärme an das "warme" Ende des sich jeweils anschließenden Wärmerohrs ab, das dann die Verlustwärme weiter nach proximal transportiert.

In einer weiteren bevorzugten Ausgestaltung der vorstehend genannten Maßnahmen ist das Wärmekoppelelement flexibel.

Hierbei ist von Vorteil, dass eine "Gelenkigkeit" der Kaskadenanordnung der einzelnen Wärmerohre bei gleichzeitig sehr guter Wärmeübertragung zwischen den einzelnen Wärmerohren erreicht wird, so dass sich diese Ausgestaltung insbesondere für Endoskope mit semi-flexiblen Schäften eignet. Ein solches flexibles Wärmekoppelelement kann beispielsweise in Form eines oder mehrerer flexibler Drähte, die beispielsweise aus Kupfer gefertigt sind, ausgeführt sein.

Weiterhin ist es bevorzugt, wenn der Drehantrieb eine Magnetkupplung aufweist, die ein außen am Schaft angeordnetes antreibendes Stellelement und ein im Schaft angeordnetes, mit dem Stellelement magnetisch zusammenwirkendes angetriebenes Element aufweist, mit dem das zumindest eine zweite Wärmerohr drehfest gekoppelt ist.

Wenn der Drehantrieb distalseitig vom Kopfstück angeordnet ist, ist das zumindest eine zweite Wärmerohr vorzugsweise durch das angetriebene Element hindurchgeführt, so dass das proximale Ende des zumindest einen zweiten Wärmerohrs in den Wärmesenkekörper greift. Das angetriebene Element ist dabei vorzugsweise ein magnetisch wirkender Ring, mit dem das zumindest eine zweite Wärmerohr drehfest gekoppelt ist. Das distale Ende des zumindest einen zweiten Wärmerohrs ist dabei vorzugsweise über eine Welle, insbesondere eine flexible Welle, mit der Kamera drehfest gekoppelt.

Im Zusammenhang mit einer der oben genannten Ausgestaltungen, wonach das erste Wärmerohr und das zumindest eine zweite Wärmerohr in dem Zwischenbereich mittels eines Wärmekoppelelements miteinander thermisch leitend verbunden sind, ist es in einer weiteren bevorzugten Ausgestaltung vorgesehen, dass das zumindest eine zweite Wärmerohr relativ zu dem Wärmekoppelelement drehbar ist.

Da die zumindest eine Lichtquelle im distalen Bereich des Schafts üblicherweise drehfest zum Schaft angeordnet ist, und entsprechend auch das erste Wärmerohr, ergibt sich durch die vorstehend genannte Maßnahme eine konstruktiv einfache Ausgestaltung, mit der das zumindest eine zweite Wärmerohr einerseits ein Drehmoment vom Drehantrieb auf die Kamera übertragen kann, zum anderen wird über das Wärmekoppelelement eine gute wärmeleitende Verbindung zwischen dem ersten Wärmerohr und dem zumindest einen zweiten Wärmerohr gewährleistet. Das Wärmekoppelelement dient somit dem zumindest einen zweiten Wärmerohr als Drehlager, wobei zur Gewährleistung einer guten Wärmeübertragung vom Wärmekoppelelement auf das zumindest eine zweite Wärmerohr Luftspalte durch ein entsprechendes Wärmekoppelmedium (bspw. ein Gel) vermieden werden können. Ebenso ist das zweite Wärmerohr relativ zum Wärmesenkekörper drehbar angeordnet, beispielsweise in diesem drehbar gelagert, während der Wärmesenkekörper drehfest im Kopfstück angeordnet ist.

In einer weiteren bevorzugten Ausgestaltung sind mit der zumindest einen Lichtquelle zumindest zwei Wärmerohre jeweils in Form einer Heatpipe thermisch gekoppelt.

Bei dieser Ausführungsform ist die Wärmeabfuhr von der zumindest einen Lichtquelle durch das Vorsehen zumindest zweier Wärmerohre, die mit der zumindest einen Lichtquelle gekoppelt sind, weiter verbessert. Die zumindest zwei Wärmerohre können dabei von der zumindest einen Lichtquelle bis zu dem Wärmesenkekörper im Kopfstück reichen, oder die einzelnen Wärmerohre erstrecken sich von der zumindest einen Lichtquelle bis in einen Zwischenbereich des Schafts, wo sie dann mit dem zumindest einen zweiten Wärmerohr thermisch gekoppelt sind, während nur das zweite Wärmerohr bis zu dem Wärmesenkekörper im Kopfstück reicht. Letztere Ausgestaltung eignet sich insbesondere wieder für eine zumindest semi-flexible Ausgestaltung des Schafts des Endoskops, und insbesondere auch für ein Videoendoskop mit einer drehbaren Kamera.

Die vorstehend genannte Ausgestaltung umfasst auch den Fall, dass im distalen Bereich des Schafts des Endoskops zwei oder mehr Lichtquellen angeordnet sind, und mit diesen Lichtquellen insgesamt zwei oder mehr Wärmerohre thermisch gekoppelt sind, wobei es sich versteht, dass die Anzahl an Lichtquellen mit der Anzahl an Wärmerohren nicht übereinstimmen muss. So ist es möglich, dass das Endoskop zwei oder mehr Lichtquellen und nur ein Wärmerohr oder nur eine Lichtquelle und zwei oder mehr Wärmerohre aufweist, das bzw. die mit der Lichtquelle bzw. den Lichtquellen thermisch gekoppelt sind.

Im Zusammenhang mit der zuvor beschriebenen Ausgestaltung ist es weiterhin bevorzugt, wenn die zumindest zwei Wärmerohre gemeinsam über das Wärmekoppelelement mit der zumindest einen Lichtquelle thermisch gekoppelt sind.

Hierbei ist von Vorteil, dass zur Kopplung der Wärmerohre mit der oder den Lichtquellen nur ein Wärmekoppelelement vorhanden ist, wodurch die Zahl der Teile des Kühlsystems und der Herstellungsaufwand des Endoskops vorteilhafterweise verringert sind.

Weitere Merkmale und Vorteile ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: ein erstes Ausführungsbeispiel eines Endoskops in einer Seitenansicht;
- Fig. 2: einen Längsschnitt durch das Endoskop in Fig. 1 entlang einer Linie II-II in Fig. 1, wobei ein Schaft des Endoskops ausschnittsweise vergrößert dargestellt ist;
- Fig. 3: das Endoskop in Fig. 1 ohne Schaft und ohne Gehäuse eines Kopfstücks in einer perspektivischen Darstellung;
- Fig. 4: eine vergrößerte Darstellung des Ausschnitts IV in Fig. 2;
- Fig. 5: eine vergrößerte Darstellung des Ausschnitts V in Fig. 2;
- Fig. 6: eine vergrößerte Darstellung des Ausschnitts VI in Fig. 3;
- Fig. 7: ein weiteres Ausführungsbeispiel eines Endoskops in einer Seitenansicht;
- Fig. 8: einen Längsschnitt durch das Endoskop entlang der Linie VIII-VIII in Fig. 7;
- Fig. 9: das Endoskop in Fig. 7 ohne Schaft und ohne Gehäuse eines Kopfstücks in einer perspektivischen Darstellung;
- Fig. 10: eine vergrößerte Darstellung des Ausschnitts X in Fig. 8;
- Fig. 11: eine vergrößerte Darstellung des Ausschnitts XI in Fig. 8;
- Fig. 12: eine vergrößerte Darstellung des Ausschnitts XII in Fig. 8;
- Fig. 13: eine vergrößerte Darstellung des Ausschnitts XIII in Fig. 9;
- Fig. 14: eine vergrößerte Darstellung des Ausschnitts XIV in Fig. 9; und
- Fig. 15: eine vergrößerte Darstellung des Ausschnitts XV in Fig. 9.

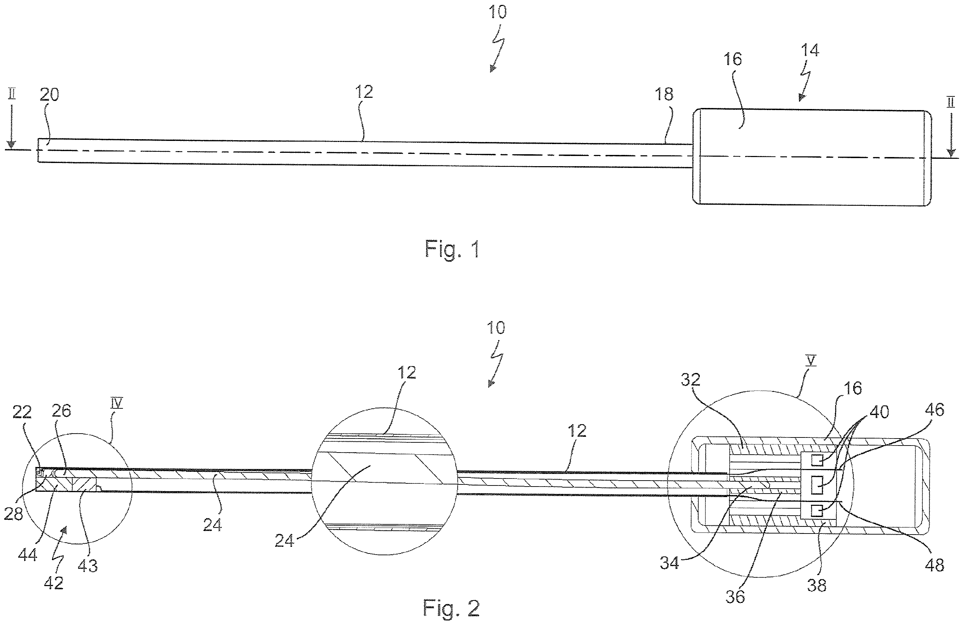

In Fig. 1 ist ein mit dem allgemeinen Bezugszeichen 10 versehenes Endoskop dargestellt, insbesondere ein medizinisches Videoendoskop.

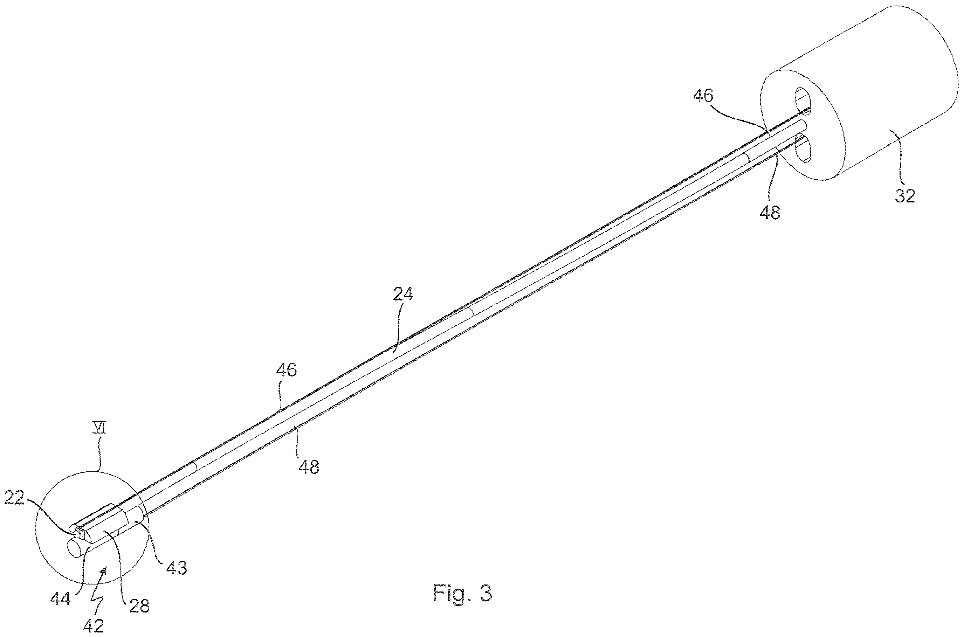

In Fig. 2 bis 6 sind weitere Einzelheiten des Endoskops 10 dargestellt, auf die nachfolgend ebenfalls Bezug genommen wird.

Das Endoskop 10 weist allgemein einen langerstreckten Schaft 12 und ein Kopfstück 14 mit einem Gehäuse 16 an einem proximalen Ende 18 des Schafts 12 auf. Ein distales Ende des Schafts 12 ist mit dem Bezugszeichen 20 in Fig. 1 versehen.

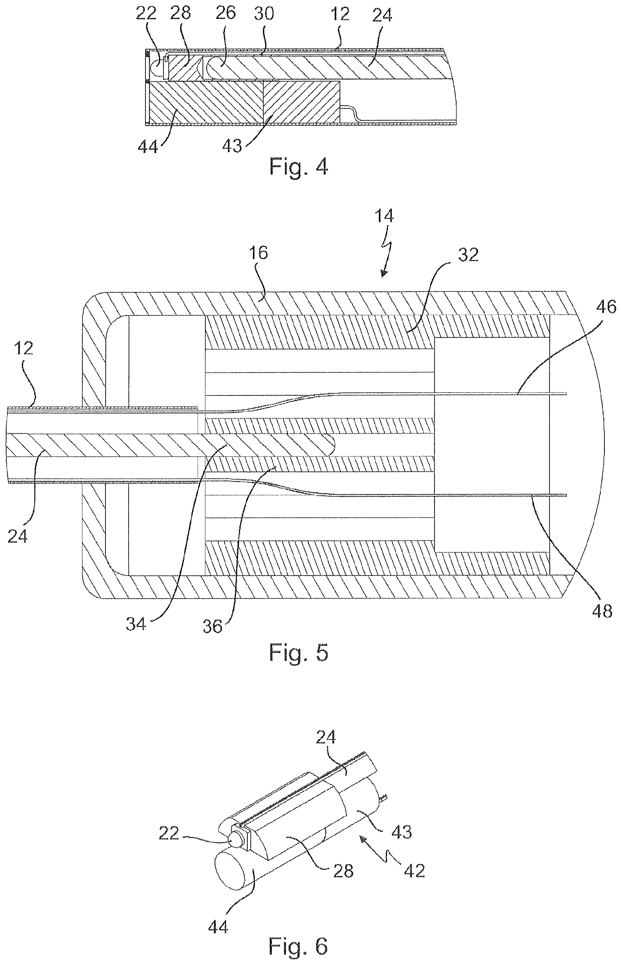

In einem distalen Bereich des Schafts 12, der insbesondere in Fig. 4 und 6 in Alleinstellung gezeigt ist, ist eine Lichtquelle 22 angeordnet, die vorzugsweise als hochleistungsfähige lichtemittierende Diode (LED oder OLED) ausgebildet ist. Im Betrieb erzeugt die Lichtquelle 22 Verlustwärme.

Um die Verlustwärme von der Lichtquelle 22 abzuführen, um zu vermeiden, dass sich der Schaft 12 insbesondere im Bereich seines distalen Endes 20 über die maximal zulässige Temperatur von 41°C gemäß Medizinproduktegesetz erwärmt, weist das Endoskop 10 eine passive Kühlung auf, die ein in dem Schaft 12 angeordnetes Wärmerohr 24 in Form einer Heatpipe aufweist.

Das Wärmerohr 24 weist ein distales Ende 26 auf, das mit der Lichtquelle 22 thermisch gekoppelt ist. Die thermische Kopplung erfolgt über ein Wärmekoppelelement 28, das als Festkörper aus einem gut wärmeleitfähigen Material, beispielsweise Kupfer oder Aluminium, gefertigt ist.

Das Wärmekoppelelement 28 weist einen hülsenartigen Fortsatz 30 auf, wie in Fig. 4 dargestellt ist, der das distale Ende 26 des Wärmerohrs 24 luftspaltfrei aufnimmt, um für einen besonders guten Wärmeübertrag vom Wärmekoppelelement 28 auf das Wärmerohr 24 zu sorgen. Das Wärmekoppelelement 28 ist seinerseits flächig mit der Rückseite der Lichtquelle 22 wärmeleitend verbunden.

Das Wärmerohr 24 erstreckt sich gemäß Fig. 2 durch den Schaft 12 hindurch bis in das Kopfstück 14, d.h. es ragt in das Gehäuse 16 des Kopfstücks 14 hinein. In dem Kopfstück 14 ist gemäß Fig. 2, 3 und 5 ein Wärmesenkekörper 32 angeordnet, mit dem das Wärmerohr 24 thermisch gekoppelt ist. Dazu ist ein proximales Ende 34 des Wärmerohrs 24 mit dem Wärmesenkekörper 32 in einer Innenhülse 36 des Wärmesenkekörpers 32 mit dieser wärmeleitend verbunden. Der Wärmesenkekörper 32 seinerseits ist mit dem Gehäuse 16 flächig wärmeleitend verbunden, so dass der Wärmesenkekörper 32 die von dem Wärmerohr 24 aufgenommene Wärme an das Gehäuse 16 des Kopfstücks 14 übertragen kann, und die Wärme wird dann vom Kopfstück 14 an die Umgebung abgestrahlt. Der Wärmesenkekörper 32 ist rohrförmig oder stabförmig ausgebildet und am Außenumfang großflächig ausgebildet, ebenso ist das Kopfstück 14 großflächig ausgebildet. Der Außendurchmesser des Wärmesenkekörpers 32 und des Gehäuses 16 des Kopfstücks sind größer als der Außendurchmesser des Schafts 12. Der Wärmesenkekörper 32 kann auch selbst Teil des Gehäuses 16 sein und die aufgenommene Wärme dann direkt an die Umgebung abgeben.

Im Einsatz des Endoskops 10 in einem chirurgischen Eingriff befindet sich das Kopfstück 14 außerhalb des menschlichen oder tierischen Körpers, so dass die Wärmeabstrahlung über das Kopfstück 14 an die Umgebung unschädlich ist. Demgegenüber bleibt der Schaft 12 außenseitig zumindest in dem Bereich, der beim chirurgischen Eingriff in den menschlichen oder tierischen Körper eingeführt ist, unterhalb der zulässigen Maximaltemperatur.

In dem Wärmesenkekörper 32 sind des Weiteren in einem durch einen hülsenartigen Fortsatz 38 gebildeten Hohlraum des Wärmesenkekörpers 32 elektronische und/oder elektrische Bauelemente 40 angeordnet, deren Verlustwärme von dem Wärmesenkekörper 32 ebenfalls an das Gehäuse 16 übertragen wird.

Das Wärmerohr 24 ist des Weiteren zumindest im Bereich des distalen Endes 20, vorzugsweise bis zum Kopfstück 14, gegen die Innenseite des Schafts 12 thermisch isoliert. Dies kann durch eine isolierende Ummantelung des Wärmerohrs 24 selbst oder durch eine Isolation auf der Innenseite des Schafts 12 realisiert sein, wie später noch mit Bezug auf ein weiteres Ausführungsbeispiel beschrieben wird.

Das Endoskop 10 weist des Weiteren eine Kamera 42 auf, die bspw. einen Bildsensor 43 auf CCD- oder CMOS-Basis aufweist, die im distalen Bereich des Schafts 12 angeordnet ist. Dem Bildsensor 43 ist lichteintrittsseitig ein Objektiv 44 vorgeordnet.

Für die Lichtquelle 22 ist eine elektrische Leitung 46 und für den Bildsensor 43 eine elektrische Leitung 48 vorgesehen, die sich von der Lichtquelle 22 bzw. von dem Bildsensor 43 bis in das Kopfstück 14 erstrecken, wie in Fig. 2 und 5 dargestellt ist. Die elektrischen Leitungen 46 und 48 führen zu entsprechenden elektrischen Anschlüssen (nicht dargestellt) am proximalen Ende des Kopfstücks 14.

Wie bereits erwähnt, ist das Wärmerohr 24 in Form einer Heatpipe ausgebildet. In dem Wärmerohr 24 sind entsprechend Kapillaren und ein Wärmeträgermedium vorhanden, wobei das Wärmeträgermedium durch die von der Lichtquelle 22 aufgenommene Verlustwärme am distalen Ende verdampft und am proximalen Ende 34 des Wärmerohrs 24 wieder kondensiert, von wo es aufgrund der Kapillarwirkung wieder zum distalen Ende gelangt. Das Wärmeträgermedium speichert Wärme somit in Form von latenter Wärme, die nach proximal transportiert und dort wieder in Form von latenter Wärme abgegeben wird. Die Wärmeübertragungsfähigkeit einer Heatpipe ist um ein Zig-faches höher als die Wärmetransportfähigkeit beispielsweise eines Kupferdrahtes.

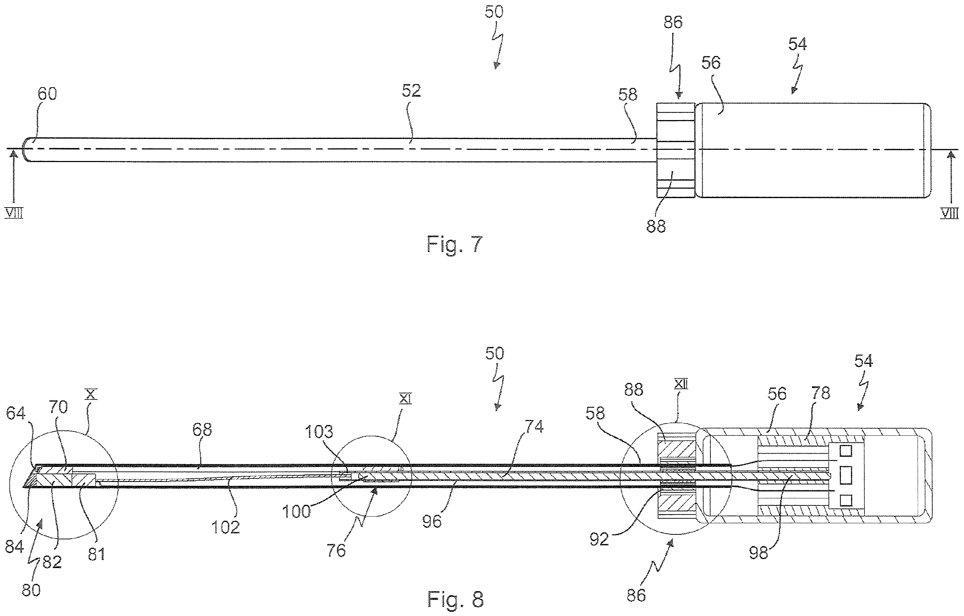

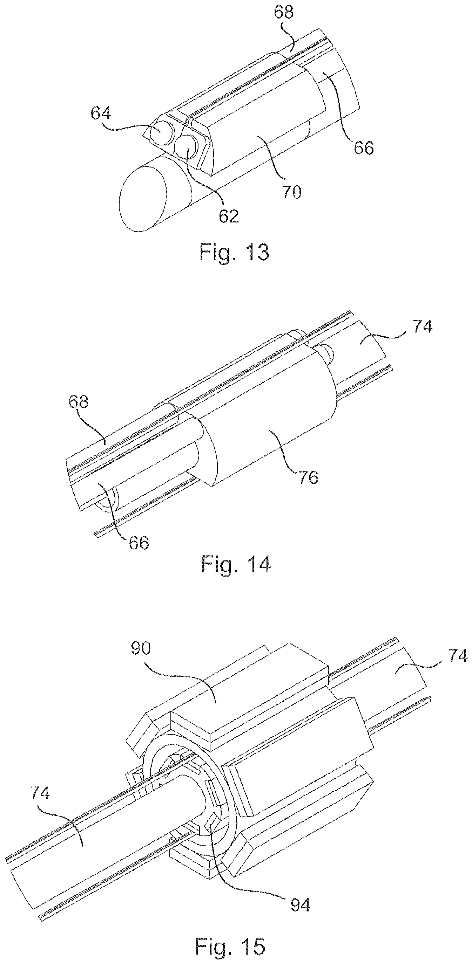

Mit Bezug auf Fig. 7 bis 15 wird nachfolgend ein weiteres Ausführungsbeispiel eines mit dem allgemeinen Bezugszeichen 50 versehenen Endoskops beschrieben. Das Endoskop 50 ist insbesondere ein medizinisches Videoendoskop.

Das Endoskop 50 weist allgemein einen Schaft 52 und ein Kopfstück 54 mit einem Gehäuse 56 auf. Das Kopfstück 54 ist an einem proximalen Ende 58 des Schafts 52 angeordnet. Ein distales Ende des Schafts 52 ist mit dem Bezugszeichen 60 versehen.

Im distalen Bereich des Schafts 52 sind eine erste Lichtquelle 62 und eine zweite Lichtquelle 64 angeordnet. Die Lichtquellen 62 und 64 sind als LEDs bzw. OLEDs ausgebildet. Die Lichtquellen 62 und 64 erzeugen im Betrieb Verlustwärme, wie bereits mit Bezug auf das vorherige Ausführungsbeispiel beschrieben wurde. Zur Abfuhr der Verlustwärme und zur Vermeidung einer Erwärmung des Schafts 52 über das zulässige Maß hinaus weist das Endoskop 50 ein erstes Wärmerohr 66, das mit den Lichtquellen 62 und 64 thermisch gekoppelt ist, und ein weiteres Wärmerohr 68 auf, das mit den Lichtquellen 62 und 64 thermisch gekoppelt ist. Für die thermische Kopplung zwischen den Wärmerohren 66 und 68 und den Lichtquellen 62 und 64 ist ein Wärmekoppelelement 70 vorhanden, in das von proximal die distalen Enden der Wärmerohre 66 und 68 möglichst luftspaltfrei eingesetzt sind, wobei das Wärmekoppelelement 70 seinerseits mit den Lichtquellen 62 und 64 wärmeleitend verbunden ist. Das Wärmekoppelelement 70 ist hier wiederum als Festkörper ausgebildet, der aus einem gut wärmeleitfähigen Material, beispielsweise Kupfer oder Aluminium, gefertigt ist.

Die Wärmerohre 66 und 68 sind jeweils als Heatpipe ausgebildet. Das erste Wärmerohr 66 und das weitere Wärmerohr 68 erstrecken sich bis in einen Zwischenbereich 72 (vgl. Fig. 8, 9 und 11), der sich zwischen dem distalen Ende 60 und dem proximalen Ende 58 des Schafts 52 befindet. Dabei ist es nicht erforderlich, dass sich der Zwischenbereich 72 in der Mitte zwischen dem distalen Ende 60 und dem proximalen Ende 58 befindet, sondern kann auch an anderen Stellen als dargestellt angeordnet sein.

In dem Zwischenbereich 72 sind das erste Wärmerohr 66 und das weitere Wärmerohr 68 mit einem zweiten Wärmerohr 74 thermisch gekoppelt. Dazu ist ein weiteres Wärmekoppelelement 76 im Zwischenbereich 72 angeordnet, das sowohl mit den proximalen Enden des ersten Wärmerohrs 66 und des weiteren Wärmerohrs 68 thermisch leitend verbunden ist, als auch mit dem distalen Ende des zweiten Wärmerohrs 74. Das Wärmekoppelelement 76 umgibt dabei die proximalen Enden der Wärmerohre 66 und 68 einerseits und das distale Ende des zweiten Wärmerohrs 74 andererseits. Auf diese Weise wird wiederum eine gute Wärmeübertragung von den Wärmerohren 66 und 68 auf das zweite Wärmerohr 74 gewährleistet.

Dabei kann vorgesehen sein, dass das Wärmekoppelelement 76 selbst flexibel ausgebildet ist, so dass die kaskadenförmige Anordnung aus den Wärmerohren 66, 68 einerseits und dem zweiten Wärmerohr 74 andererseits im Zwischenbereich 72 eine gewisse Gelenkigkeit besitzt, so dass der Schaft 52 des Endoskops 50 insbesondere auch semi-flexibel ausgestaltet sein kann.

Das zweite Wärmerohr 74 erstreckt sich nun bis in das Kopfstück 54, in dem es mit einem Wärmesenkekörper 78 thermisch gekoppelt ist, wobei hinsichtlich der thermischen Kopplung und der Ausgestaltung des Wärmesenkekörpers 78 auf die Beschreibung des Wärmesenkekörpers 32 des vorherigen Ausführungsbeispiels verwiesen wird, wobei im Wärmesenkekörper 78 wiederum elektronische und/oder elektrische Bauelemente angeordnet sein können.

Das Endoskop 50 weist weiterhin eine im distalen Bereich des Schafts 52 angeordnete Kamera 80 auf, die bspw. einen Bildsensor 81 in CCD- oder CMOS-Ausführung aufweist. Distalseitig des Bildsensors 81 weist die Kamera 80 ein Objektiv 82 auf. Das Objektiv 82 weist weiterhin eine Schrägblickoptik 84 auf, so dass die Blickrichtung des Endoskops 50 mit der Längsachse des Schafts 52 einen Winkel von ≠ 0°, beispielsweise 30° einschließt. Die Schrägblickoptik 84 kann auch in das Objektiv 82 integriert sein.

Durch Drehen des Schafts 52 um seine Längsachse ermöglicht das Endoskop 50 bei gleicher Orientierung der Längsachse des Schafts 52 unterschiedliche Blickrichtungen des Endoskops 50 im beobachteten Areal. Um dem das Endoskop 50 bedienenden Arzt die Orientierung auf dem Videomonitor (nicht dargestellt), auf dem das Endoskopbild visuell dargestellt wird, zu erleichtern, ist es bei dem Endoskop 50 vorgesehen, dass die Kamera 80, hier nur der Bildsensor 81 der Kamera 80, relativ zum Schaft 52 um die optische Achse der Kamera 80 verdrehbar ist.

Für die Kamera 80 (Bildsensor 81) ist am Kopfstück 54 entsprechend ein Drehantrieb 86 vorhanden, der in Form einer Magnetkupplung ausgebildet ist.

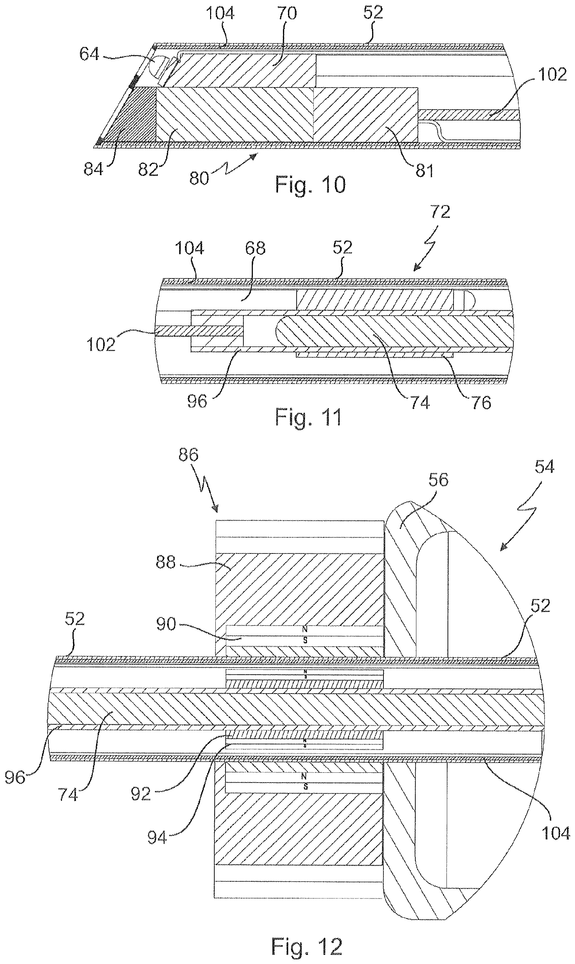

Der Drehantrieb 86 weist ein außen am Schaft 52 am proximalen Ende 58 angeordnetes antreibendes Stellelement 88 auf, das als Handrad ausgebildet ist. Das Stellelement 88 weist an seinem Innenumfang eine Mehrzahl an Magneten 90 auf. In Fig. 12 sind die Magnetpole mit N und S veranschaulicht.

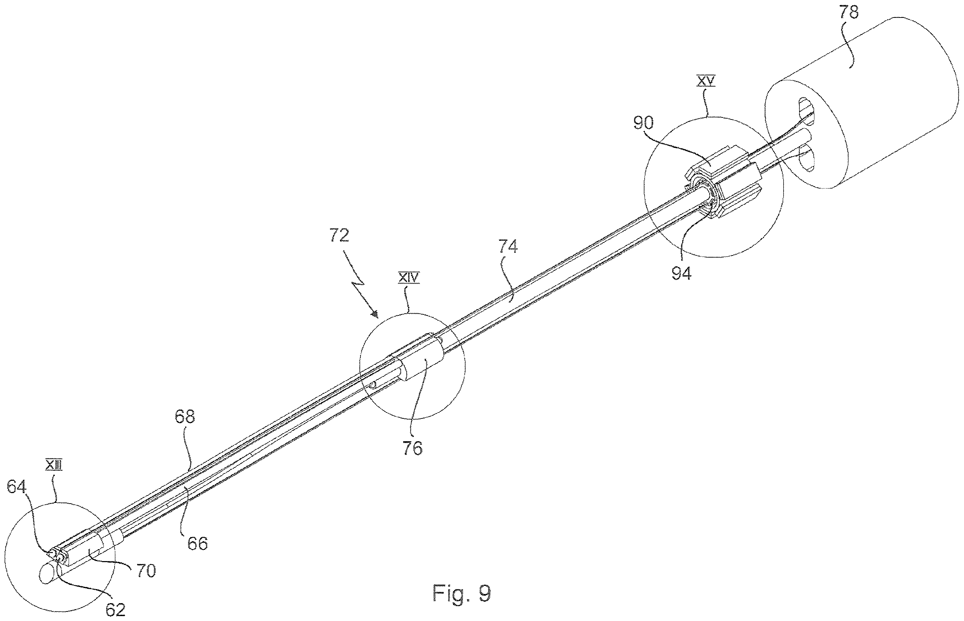

Der Drehantrieb 86 weist weiterhin ein mit dem Stellelement 88 magnetisch zusammenwirkendes angetriebenes Element 92 auf, das ebenfalls eine Mehrzahl an Magneten 94 (vgl. Fig. 9) aufweist, deren Pole in Fig. 12 ebenfalls mit N und S veranschaulicht sind. Aufgrund des magnetischen Kraftschlusses zwischen den Magneten 90 des Stellelements 88 und dem Magneten 94 des angetriebenen Elements 92 folgt das angetriebene Element 92 einer Drehbewegung des Stellelements 88 in gleicher Drehrichtung.

Das zweite Wärmerohr 74 ist dabei mit dem angetriebenen Element 92, durch dieses hindurchgeführt, drehfest gekoppelt, so dass eine Drehung des angetriebenen Elements 92 um seine Längsachse eine Drehung des zweiten Wärmerohrs 74 in gleicher Drehrichtung bewirkt. Das zweite Wärmerohr 74 ist ebenfalls relativ zum Schaft 52 um seine Längsachse drehbar in dem Schaft aufgenommen. Das zweite Wärmerohr 74 dient in diesem Ausführungsbeispiel somit nicht nur zum Wärmetransport der Verlustwärme der Lichtquellen 62 und 64 in das Kopfstück 54, sondern auch als Drehmomentübertragungselement für die Kamera 80.

Um das Wärmerohr 74 in Form der Heatpipe gegen mechanische Belastungen bei der Drehmomentübertragung besser zu schützen, ist das zweite Wärmerohr 74 von einem Rohr 96 umgeben, das beispielsweise aus Kupfer oder Aluminium, allgemein aus einem thermisch gut leitenden Material gefertigt ist.

Das zweite Wärmerohr 74 ist in dem Zwischenbereich 72 in dem Wärmekoppelelement 76 drehbar gelagert, während das Wärmekoppelelement 76 selbst drehfest wie das erste Wärmerohr 66 und das zweite Wärmerohr 68 und die Lichtquellen 62 und 64 gehalten ist.

Die drehbare Lagerung des zweiten Wärmerohrs 74 in dem Wärmekoppelelement 76 ist dabei so realisiert, dass weiterhin eine gute Wärmeübertragung von den Wärmerohren 66 und 68 auf das zweite Wärmerohr 74 gewährleistet ist.

Ein proximales Ende 98 des zweiten Wärmerohrs 74 ist ebenso drehbar in dem Wärmesenkekörper 78 im Kopfstück 54 gelagert.

Von einem distalen Ende 100 des zweiten Wärmerohrs 74 erstreckt sich nach distal ein Drehmomentübertragungselement 102, das hier als flexible Welle ausgebildet ist, was insbesondere dann vorteilhaft ist, wenn die Drehachse der Kamera 80 nicht mit der Längsachse des zweiten Wärmerohrs 74 fluchtet, wie insbesondere aus Fig. 8 hervorgeht.

Das Drehmomentübertragungselement 102 ist hier mit einem distalen Ende 103 des Rohrs 96 drehfest gekoppelt.

Um bei dem Endoskop 50 die Blickrichtung zu verändern, wird der Schaft 52 um seine Längsachse gedreht, und um dabei die Orientierung der Kamera 80 nicht zu verändern, wird das Stellelement 88 festgehalten, wodurch über das angetriebene Element 92 entsprechend das zweite Wärmerohr 74 und über das Drehmomentübertragungselement 102 die Kamera 80 ebenfalls festgehalten wird, wodurch die Kamera 80 an einer Drehung um ihre eigene optische Achse gehindert wird, so dass sich der Schaft 52 um die Kamera 80 herum dreht.

Wie bei dem vorhergehenden Ausführungsbeispiel ist der Schaft 52 gegen die Wärmerohre 66, 68 und 74 thermisch isoliert. Hierzu ist der Schaft 52 innenseitig mit einer thermischen Isolierung 104 versehen, die in Form eines Zwischenrohrs aus einem wärmedämmenden Material ausgebildet ist. Die thermische Isolierung 104 erstreckt sich hier vom distalen Ende des Schafts 52 bis zum proximalen Ende des Schafts, der teilweise in das Kopfstück 54 hineinragt, wie in Fig. 8 und 12 dargestellt ist.

Alternativ oder zusätzlich können auch die Wärmerohre 66, 68 und 74 außenseitig mit einer thermischen Isolierung (nicht dargestellt) umhüllt sein.

Es versteht sich, dass bei dem Ausführungsbeispiel gemäß Fig. 7 bis 15 auch nur eine Lichtquelle, beispielsweise die Lichtquelle 64 vorgesehen sein kann, wobei dann nur das Wärmerohr 68 im Zwischenbereich 72 mit dem zweiten Wärmerohr 74 thermisch gekoppelt ist, während die übrige Ausgestaltung, insbesondere die Drehbarkeit des zweiten Wärmerohrs 74 relativ zum Schaft 52 erhalten bleibt.

Umgekehrt kann das Endoskop 50 auch mehr als die zwei Lichtquellen 62 und 64 und auch eine größere Anzahl an Wärmerohren 66, 68 aufweisen, wobei allerdings im Hinblick auf den möglicherweise sehr kleinen Durchmesser des Schafts 52 eine Anzahl von zwei Wärmerohren 66, 68 bei zwei Lichtquellen 62 und 64 vorteilhaft ist.

Des Weiteren kann die Wärmerohrkaskade aus erstem Wärmerohr 66, weiterem Wärmerohr 68 und zweitem Wärmerohr 74 anstatt wie hier zweistufig auch drei-, vier-, usw. -stufig ausgebildet sein.

## Patentansprüche

1. Endoskop, mit einem langerstreckten Schaft (12; 52), mit einem Kopfstück (14; 54) an einem proximalen Ende (18; 58) des Schafts (12; 52), wobei das Kopfstück (14; 54) ein Gehäuse (16; 56) aufweist, mit zumindest einer in dem Schaft (12; 52) in einem distalen Bereich desselben angeordneten Lichtquelle (22; 62, 64), die Verlustwärme erzeugt, und mit einer passiven Kühlung, die zumindest ein in dem Schaft (12; 52) angeordnetes Wärmerohr (24; 66, 68, 74) in Form einer Heatpipe aufweist, die mit der zumindest einen Lichtquelle (22; 62, 64) thermisch gekoppelt ist, um die Verlustwärme in Richtung nach proximal abzuführen, wobei sich das zumindest eine Wärmerohr (24; 66, 68, 74) bis in das Kopfstück (14; 54) erstreckt, und wobei in dem Kopfstück (14; 54) ein Wärmesenkekörper (32; 78) angeordnet ist, mit dem das zumindest eine Wärmerohr (24; 66, 68, 74) thermisch gekoppelt ist, und der die Verlustwärme von dem zumindest einen Wärmerohr (24; 66, 68, 74) aufnimmt und direkt oder über das Gehäuse (16; 56) des Kopfstücks (14; 54) an die Umgebung abgibt, **dadurch gekennzeichnet, dass** das zumindest eine Wärmerohr (66, 68) ein erstes Wärmerohr (66, 68), das mit der zumindest einen Lichtquelle (62, 64) gekoppelt ist und sich bis zu einem Zwischenbereich (72) im Schaft (52) zwischen dem distalen und proximalen Ende (60, 58) desselben erstreckt, und zumindest ein zweites Wärmerohr (74) in Form einer Heatpipe aufweist, das mit dem ersten Wärmerohr (66, 68) thermisch gekoppelt ist, und das sich von dem Zwischenbereich (72) bis in das Kopfstück (54) erstreckt und mit dem Wärmesenkekörper (78) thermisch gekoppelt ist, und dass das zumindest eine zweite Wärmerohr (74) relativ zum Schaft (52) um eine Längsachse des zumindest einen zweiten Wärmerohrs (74) drehbar ist, und dass das zumindest eine zweite Wärmerohr (74) einerseits mit einer im distalen Bereich des Schafts (52) angeordneten Kamera (80) verbunden und andererseits mit einem Drehantrieb (86) verbunden ist, um eine Drehung der Kamera (80) relativ um ihre eigene optische Achse relativ zum Schaft (52) zu ermöglichen.

2. Endoskop nach Anspruch 1, **dadurch gekennzeichnet, dass** der Schaft (12; 52) zumindest im Bereich seines distalen Endes innenseitig gegen das zumindest eine Wärmerohr (24; 66, 68, 74) thermisch isoliert ist.

3. Endoskop nach Anspruch 1 oder 2, **dadurch gekennzeichnet, dass** elektronische und/oder elektrische Bauelemente (40) in einem Hohlraum des Wärmesenkekörpers (32; 78) angeordnet sind.

4. Endoskop nach einem der Ansprüche 1 bis 3, **dadurch gekennzeichnet, dass** ein distales Ende (26) des zumindest einen Wärmerohrs (24; 66, 68) mit der zumindest einen Lichtquelle (22; 62, 64) mittels eines Wärmekoppelelements (28; 70) thermisch leitend verbunden ist.

5. Endoskop nach einem der Ansprüche 1 bis 4, **dadurch gekennzeichnet, dass** das der Wärmesenkekörper (32; 78) einen proximalen Endbereich des zumindest einen Wärmerohrs (24; 74) umgibt und mit diesem unmittelbar in Berührung steht.

6. Endoskop nach einem der Ansprüche 1 bis 5, **dadurch gekennzeichnet, dass** sich das erste Wärmerohr (66, 68) und das zumindest eine zweite Wärmerohr (74) in dem Zwischenbereich (72) in Längsrichtung des Schafts (52) teilweise überlappen.

7. Endoskop nach einem der Ansprüche 1 bis 6, **dadurch gekennzeichnet, dass** das erste Wärmerohr (66, 68) und das zumindest eine zweite Wärmerohr (74) in dem Zwischenbereich (72) mittels eines Wärmekoppelelements (76) miteinander thermisch leitend verbunden sind, das das erste und das zumindest eine zweite Wärmerohr (66, 68, 74) umgibt.

8. Endoskop nach Anspruch 7, **dadurch gekennzeichnet, dass** das Wärmekoppelelement (76) flexibel ist.

9. Endoskop nach einem der Ansprüche 1 bis 8, **dadurch gekennzeichnet, dass** der Drehantrieb (86) eine Magnetkupplung aufweist, die ein außen am Schaft (52) angeordnetes antreibendes Stellelement (88) und ein im Schaft (52) angeordnetes, mit dem Stellelement (88) magnetisch zusammenwirkendes angetriebenes Element (92) aufweist, mit dem das zumindest eine zweite Wärmerohr (74) drehfest gekoppelt ist.

10. Endoskop nach Anspruch7 oder 8, **dadurch gekennzeichnet, dass** das zumindest eine zweite Wärmerohr (74) relativ zu dem Wärmekoppelelement (76) drehbar ist.

11. Endoskop nach einem der Ansprüche 1 bis 10, **dadurch gekennzeichnet, dass** mit der zumindest einen Lichtquelle (22; 62, 64) zumindest zwei Wärmerohre (66, 68) jeweils in Form einer Heatpipe thermisch gekoppelt sind.

12. Endoskop nach Anspruch 4 und nach Anspruch 11, **dadurch gekennzeichnet, dass** die zumindest zwei Wärmerohre (66, 68) gemeinsam über das Wärmekoppelelement (70) mit der zumindest einen Lichtquelle thermisch gekoppelt sind.

13. Endoskop nach Anspruch 11 oder 12, **dadurch gekennzeichnet, dass** die zumindest zwei Wärmerohre (66, 68) mit dem zumindest einen zweiten Wärmerohr (74) in dem Zwischenbereich (72) thermisch gekoppelt sind.

## Claims

1. Endoscope, comprising an elongated shaft (12; 52), a headpiece (14; 54) at a proximal end (18; 58) of the shaft (12; 52), the headpiece (14; 54) having a housing (16; 56), at least one light source (22; 62, 64), which is arranged in the shaft (12; 52) in a distal area thereof and produces lost heat, and a passive cooling, which has at least one heat pipe (24; 66, 68, 74) which is arranged in the shaft (12; 52) and is thermally coupled to the at least one light source (22; 62, 64) in order to lead away the lost heat in proximal direction, wherein the at least one heat pipe (24; 66, 68, 74) extends into the headpiece (14; 54), and wherein a heat sink body (32; 78) is arranged in the headpiece (14; 54), to which heat sink body (32; 78) the at least one heat pipe (24; 66, 68, 74) is thermally coupled, and which absorbs the lost heat from the at least one heat pipe (24; 66, 68, 74) and emits the lost heat to the environment directly or via the housing (16; 56) of the headpiece (14; 54), **characterized in that** the at least one heat pipe (66, 68) comprises a first heat pipe (66, 68) which is coupled to the at least one light source (62, 64) and extends to an intermediate area (72) in the shaft (52) between the distal and proximal ends (60, 58) thereof, and at least one second heat pipe (74) which is thermally coupled to the first heat pipe (66, 68), which second heat pipe (74) extends from the intermediate area (72) into the headpiece (54), and is thermally coupled to the heat sink body (78), and that the at least one second heat pipe (74) can rotate relative to the shaft (52) about a longitudinal axis of the at least one second heat pipe (74), and **in that** the at least one second heat pipe (74) is connected on the one hand to a camera (80), which is arranged in the distal area of the shaft (52), and on the other hand to a rotating drive (86), in order to allow the camera (80) to rotate relative to the shaft (52) about its own optical axis.

2. Endoscope of Claim 1, **characterized in that**, at least in the area of its distal end, the shaft (12; 52) is thermally insulated on the inside from the at least one heat pipe (24; 66, 68, 74).

3. Endoscope of Claim 1 or 2, **characterized in that** electronic and/or electrical components (40) are arranged in a cavity in the heat sink body (32; 78).

4. Endoscope of any one of Claims 1 through 3, **characterized in that** a distal end (26) of the at least one heat pipe (24; 66, 68) is thermally conductively connected to the at least one light source (22; 62, 64) by means of a heat coupling element (28; 70).

5. Endoscope of any one of Claims 1 through 4, **characterized in that** the heat sink body (32; 78) surrounds a proximal end area of the at least one heat pipe (24; 74), and makes direct contact with it.

6. Endoscope of any one of Claims 1 through 5, **characterized in that** the first heat pipe (66, 68) and the at least one second heat pipe (74) partially overlap in the intermediate area (72) in the longitudinal direction of the shaft (52).

7. Endoscope of any one of Claims 1 through 6, **characterized in that** the first heat pipe (66, 68) and the at least one second heat pipe (74) are thermally conductively connected to one another in the intermediate area (72) by means of a heat coupling element (76) which surrounds the first and the at least one second heat pipe (66, 68, 74).

8. Endoscope of Claim 7, **characterized in that** the heat coupling element (76) is flexible.

9. Endoscope of any one of Claims 1 through 8, **characterized in that** the rotating drive (86) has a magnetic coupling, which has a driving actuating element (88), which is arranged externally on the shaft (52), and a driven element (92), which interacts magnetically with the actuating element (88) and is arranged in the shaft (52), and to which the at least one second heat pipe (74) is coupled in rotationally fixed manner relative to one another.

10. Endoscope of Claim 7 or 8, **characterized in that** the at least one second heat pipe (74) can rotate relative to the heat coupling element (76).

11. Endoscope of any one of Claims 1 through 10, **characterized in that** at least two heat pipes (66, 68) are thermally coupled to the at least one light source (22; 62, 64).

12. Endoscope of Claim 4 and of Claim 11, **characterized in that** the at least two heat pipes (66, 68) are jointly thermally coupled via the heat coupling element (70) to the at least one light source.

13. The endoscope of Claim 11 or 12, **characterized in that** the at least two heat pipes (66, 68) are thermally coupled to the at least one second heat pipe (74) in the intermediate area (72).

## Revendications

1. Endoscope, avec un arbre allongé (12 ; 52), avec un élément de tête (14 ; 54) à une extrémité proximale (18 ; 58) de l'arbre (12 ; 52), dans lequel l'élément de tête (14 ; 54) présente un boîtier (16 ; 56), avec au moins une source de lumière (22 ; 62, 64) agencée dans l'arbre (12 ; 52) dans une zone distale de celui-ci, qui génère de la chaleur perdue, et avec un refroidissement passif, qui présente au moins un tube de chaleur (24 ; 66, 68, 74) agencé dans l'arbre (12 ; 52) en tant que caloduc, qui est couplé thermiquement avec l'au moins une source de lumière (22 ; 62, 64) pour évacuer la chaleur perdue dans la direction proximale, dans lequel l'au moins un tube de chaleur (24 ; 66, 68, 74) s'étend jusque dans l'élément de tête (14 ; 54), et dans lequel un corps de puits de chaleur (32 ; 78) est agencé dans l'élément de tête (14 ; 54), avec lequel l'au moins un tube de chaleur (24 ; 66, 68, 74) est couplé thermiquement, et qui absorbe la chaleur perdue de l'au moins un tube de chaleur (24 ; 66, 68, 74) et la transmet à l'environnement directement ou par le biais du boîtier (16 ; 56) de l'élément de tête (14 ; 54), **caractérisé en ce que** l'au moins un tube de chaleur (66, 68) présente un premier tube de chaleur (66, 68), qui est couplé avec l'au moins une source de lumière (62, 64) et s'étend jusqu'à une zone intermédiaire (72) dans l'arbre (52) entre l'extrémité distale et proximale (60, 58) de celui-ci, et au moins un deuxième tube de chaleur (74) en tant que caloduc, qui est couplé thermiquement avec le premier tube de chaleur (66, 68), et qui s'étend de la zone intermédiaire (72) jusque dans l'élément de tête (54) et est couplé thermiquement avec le corps de puits de chaleur (78), et **en ce que** l'au moins un deuxième tube de chaleur (74) est rotatif par rapport à l'arbre (52) autour d'un axe longitudinal de l'au moins un deuxième tube de chaleur (74), et **en ce que** l'au moins un deuxième tube de chaleur (74) est relié d'une part à une caméra (80) agencée dans la zone distale de l'arbre (52) et relié d'autre part à un entraînement rotatif (86) afin de permettre une rotation de la caméra (80) par rapport à son propre axe optique par rapport à l'arbre (52).

2. Endoscope selon la revendication 1, **caractérisé en ce que** l'arbre (12 ; 52) est isolé thermiquement au moins dans la zone de son extrémité distale côté intérieur contre l'au moins un tube de chaleur (24 ; 66, 68, 74).

3. Endoscope selon la revendication 1 ou 2, **caractérisé en ce que** des composants électroniques et/ou électriques (40) sont agencés dans une cavité du corps de puits de chaleur (32 ; 78).

4. Endoscope selon l'une quelconque des revendications 1 à 3, **caractérisé en ce qu'**une extrémité distale (26) de l'au moins un tube de chaleur (24 ; 66, 68) est reliée de manière thermoconductrice à l'au moins une source de lumière (22 ; 62, 64) au moyen d'un élément de couplage de chaleur (28 ; 70).

5. Endoscope selon l'une quelconque des revendications 1 à 4, **caractérisé en ce que** le corps de puits de chaleur (32 ; 78) entoure une zone d'extrémité proximale de l'au moins un tube de chaleur (24 ; 74) et est en contact direct avec celui-ci.

6. Endoscope selon l'une quelconque des revendications 1 à 5, **caractérisé en ce que** le premier tube de chaleur (66, 68) et l'au moins un deuxième tube de chaleur (74) se chevauchent en partie dans la zone intermédiaire (72) dans le sens longitudinal de l'arbre (52).

7. Endoscope selon l'une quelconque des revendications 1 à 6, **caractérisé en ce que** le premier tube de chaleur (66, 68) et l'au moins un deuxième tube de chaleur (74) sont reliés de manière thermoconductrice l'un à l'autre dans la zone intermédiaire (72) au moyen d'un élément de couplage de chaleur (76), qui entoure le premier et l'au moins un deuxième tube de chaleur (66, 68, 74).

8. Endoscope selon la revendication 7, **caractérisé en ce que** l'élément de couplage de chaleur (76) est flexible.

9. Endoscope selon l'une quelconque des revendications 1 à 8, **caractérisé en ce que** l'entraînement rotatif (86) présente un accouplement magnétique, qui présente un élément de commande (88) entraînant agencé à l'extérieur sur l'arbre (52) et un élément (92) entraîné coopérant magnétiquement avec l'élément de commande (88), agencé dans l'arbre (52), avec lequel l'au moins un deuxième tube de chaleur (74) est couplé solidaire en rotation.

10. Endoscope selon la revendication 7 ou 8, **caractérisé en ce que** l'au moins un deuxième tube de chaleur (74) est rotatif par rapport à l'élément de couplage de chaleur (76).

11. Endoscope selon l'une quelconque des revendications 1 à 10, **caractérisé en ce qu'**au moins deux tubes de chaleur (66, 68) respectivement en tant que caloduc sont couplés thermiquement à l'au moins une source de chaleur (22 ; 62, 64).

12. Endoscope selon la revendication 4 et selon la revendication 11, **caractérisé en ce que** les au moins deux tubes de chaleur (66, 68) sont couplés thermiquement ensemble avec l'au moins une source de chaleur par le biais de l'élément de couplage de chaleur (70).

13. Endoscope selon la revendication 11 ou 12, **caractérisé en ce que** les au moins deux tubes de chaleur (66, 68) sont couplés thermiquement avec l'au moins un deuxième tube de chaleur (74) dans la zone intermédiaire (72).
